# EUROPEAN PATENT SPECIFICATION

(11) **EP 1 124 619 B1**
(45) Date of publication and mention of the grant of the patent: **05.01.2005**
(21) Application number: 99939599.9
(22) Date of filing: 26.07.1999
(51) Int. Cl.: B01D 9/00, C07C 7/14, C13F 1/06

(54) **PROCESS FOR PURIFYING SOLID CHEMICAL PRODUCTS BY DRY-SWEATING**
VERFAHREN ZUR REINGEWINNUNG FESTER CHEMISCHER PRODUKTE DURCH TROCKENES SCHWITZEN
PROCEDE DE PURIFICATION DE PRODUITS CHIMIQUES PAR RESSUAGE A SEC

(30) Priority: 29.07.1998 IT RM980505
(43) Date of publication of application: 22.08.2001
(73) Proprietor: UNIVERSITA' DEGLI STUDI DI ROMA "LA SAPIENZA", 00185 Roma (IT)
(72) Inventor: CHIANESE, Angelo, I-00189 Roma (IT)
(74) Representative: Banchetti, Marina
(86) International application number: PCT/IT1999/000236
(87) International publication number: WO 2000/006279

(56) References cited:
- EP-A- 0 492 149
- GB-A- 429 474
- GB-A- 694 804
- GB-A- 956 997
- GB-A- 1 322 020
- US-A- 4 795 571

## Description

The present invention concerns a process for purifying solid chemical products by dry-sweating. More specifically, the invention concerns a new method for purifying raw solid chemical substances obtained by crystallization from the melt, which method is based on the general technique known as sweating, allowing to obtain, at the end of the treatment, a solid of reduced mass but having a higher purity, and a liquid residue containing a high concentration of impurities expelled from the solid.

As it is known, the products obtained at the solid state in the chemical industry obtained from crystallization processes, frequently contain foreign matter incorporated therein. In particular, industrially produced crystalline substances may contain significant amounts of inclusions, this term meaning any foreign body (either in the solid, or liquid, or gaseous state) present in a solid. For instance, a material fastly crystallizing may entrap droplets of mother liquor, thus resulting in inclusions in the solid obtained, which negatively affect the solid purity.

For various causes, including, first of all, the influence of a temperature gradient in the solid, the said inclusions tend to migrate from the interior towards the surface of the solid, until they are expelled from the solid. In particular, a controlled heating of the solid mass obtained from the crystallization process, such as to result in a partial melting, allows to obtain a purification of the said solid, in an operation currently referred to as "sweating". The temperature to which the solid is to be brought is slightly above the equilibrium temperature of the pure solid in contact with the melt mixture present as an inclusion. In practice, since the presence of impurities lowers the melting temperature, sections of crystalline product with higher impurity concentration tend to melt first; melting preferentially takes place around the inclusion, thus resulting, on one hand, in an increase of the inclusion volume and on the other hand, in the formation of cracks and pores within the solid, through which the inclusions find an easy escape.

The overall sweating process occurs in two phases, the first phase including the migration of the liquid droplets present in the solid as inclusions and the partial melting of the solid, which favours such migration. Once the droplets containing the impurities have reached the solid surface, they must be removed from said surface, on which they adhere. Draining away this liquid is increasingly difficult the more the viscosity and the surface tension thereof are high; in most cases a spontaneous separation of the droplets of impure melt product from the solid surface cannot be obtained. For this reason, the sweating technique considered to be up to now the most efficient one, which has been widely employed at the industrial level, is the technique known as "wet-sweating", wherein the solid to be purified is heated by contact with a liquid at a higher temperature, specifically with the same purified product at the molten state. It is evident that the presence of a liquid or melt flowing in contact with the sweating solid enhances the draining, and, in addition, it has a real washing action on the solid surface.

An industrially applied purification process employing the wet-sweating technique is, for instance, the Tsukushima process (M. Morita, K. Nakamaru, K. Tagekami, Tsukushima Kikai Co., "Industrial continuous melt crystallization", Proceed. of World Congress II of Chemical Engineering, Tokyo, Sept. 21-25, 1986), wherein the product obtained from a cascade series of crystallizers is purified in a column equipped with a rotary blade stirrer (i.e., the purifier), by counter-current contact with the melt purified product. Specifically, the crystals to be purified are fed at the top of the column, while the melt purified product is produced at the bottom, by means of heating devices placed at the base of the column. In the first part of the process the impure crystals undergo a washing action, which removes the mother liquor residues adhered on their surfaces; thereafter, as they progress downwards, the crystals undergo a true sweating action, and they are obtained in a purified form from the bottom of the column. The impure liquid reaching the column head is recycled to the crystallizers upstream the purifying column.

A similar industrial process based on wet-sweating is the process designed by Kureha Chemical Industries Co. (J. Yamada, C. Shimizu, S. Saitoh, "Purification of organic chemicals by Kureha continuous crystal purifier", Industrial Crystallization 81, ed. S.J. Jancic, E.J. de Jong, North Holland Publ. Co., 1982), which is also covered by the European patent No. 0242181, of the same owner. Said process employs a purification column with a "8"-shaped cross-section, having a double rotary blade shaft serving as an elevator/stirrer device. In contrast to the previously disclosed case, the impure crystalline product is fed to the column from the bottom thereof and the purified melt product is obtained at the top of the column, where a heating device is placed. Apart from the opposed progression sense of the streams being treated, the purifying system is practically the same as the previous one, the crystals to be purified being contacted, counter-current, with the melt purified product, thereby undergoing first a washing phase, followed by a true sweating.

According to various more recently proposed methods, the sweating process represents the final purification stage in the procedures of crystallization from the melt carried out with the falling film technique. After the formation of a crystalline solid on the inner wall of an externally cooled tube, and after the first separation of the impure liquid left uncrystallized, by draining (crystallization step), the solid formed on the tube wall is heated again, to a lower temperature and for a shorter time, so as to result in a partial fusion of the said solid (sweating phase). A purified solid product is thus obtained, along with a comparatively less pure melt, which is collected by draining from the bottom of the tube and removed. Finally, the temperature is increased so as to obtain the melting of the purified product remaining on the tube and thus recover it. Such process, carried out in a single stage in a dynamic crystallizer of the falling film type, has been reported to afford, according to an experimental work by Jancic (S.J. Jancic, Sulzer Crystallization Systems, "Fractional crystallization", Proceed. of the 10th Symposium on Industrial Crystallization, Bechyne, Czechoslovakia, Sept. 21-25, 1987, pp. 57-70), for instance in the purification of benzoic acid, a coefficient of distribution of the impurities of 0.34 (this parameter defining the ratio between the impurity concentration in the solid after the treatment and the same concentration before the treatment). Specifically, the experimental work reports a reduction of the impurities from 1.38% in the feed to 0.47% in the crystalline layer resulting from the sweating operation, and an increase to 3.14% of the impurity concentration in the discarded residue, this value amounting to an impurity concentration in the final residue equal to 2.3 times the concentration in the solid undergoing the treatment.

The above technology, which does not appear, therefore, to be particularly efficient for a single crystallization stage, has been applied, in order to enhance its performance, to multistage fractional crystallization processes as such as the process described for the purification of bisphenol-A in the European patent No. 0475893 (Sulzer Chemtech). In the multistage crystallization and sweating process disclosed in the said document, the multistage dynamic crystallizer known as Sulzer crystallizer (U.S. Patent No. 3621664, Saxer) is preferably employed.

By means of the above technique, which is presently considered to be the most reliable one, it is possible to produce, according to the information given in the patent, bisphenol-A having a purity exceeding 99.50% and, preferably, above 99.90%. As it is evident from the description, however, such purification level can be achieved through a complex series of subsequent solidification and melting steps, in the frame of three or more stages of crystallization from the melt, and, in addition, with a remarkable energy consumption.

On the other hand, the sweating process wherein the solid is treated by means of a hot gas, referred to as to a dry-sweating, has received lower attention, up to now, from the researchers, as well as a quite limited industrial application. Several reasons substantiate the inadequacy of the said process, carried out with the techniques proposed to date. Among such reasons, firstly, the following ones should be mentioned:
- draining away the melt produced by a partial melting of the solid is a difficult operation since, as noted in the foregoing, it is extremely difficult to obtain the removal of the impure liquid droplets from the purified crystalline solid
- by means of the only action of a flowing gas and of the gravity;
- the process may lead, if it is not accurately controlled, to the formation of large cracks in the solid, which may cause the breaking thereof;
- the draining of the melt through a bed of crystal particles results in the agglomeration of the said particles.

Another purification process by crystallization from the melt falling within the "dry-sweating" definition is the process disclosed in the U.S. patent No. 5447542 (Hoechst). According to such process, an inert gas is first introduced into the melt, the melt is then put under pressure and cooled, so as to induce the solidification, and then the residual melt is separated from the solid; thereafter, the pressure is lowered in order to cause the degassation of the solid and, at the same time, obtain a partial melting or sweating of the same solid. The pressure reduction, thus, brings about the formation of a sweating oil with a high concentration of impurities. A good separation is obtained, for instance, for naphthalene/biphenyl mixtures, p-/m-dichlorobenzene mixtures, etc.. However, no examples are disclosed of an industrial-scale process showing, specifically, how the sweat oil is drained away from the solid.

A type of process based on dry-sweating which, on the contrary, has been developed at the industrial scale is the process disclosed in the European patent No. 0492149 (Santrade Ltd.). According to said process, solid particles, for instance in the form of pastilles, are produced starting from a melt, by applying the known technique of the cooled steel belts by Sandvik, and the particles thus produced are then dropped from the top of a vessel with vertical axis and square cross-section, counter-current with a hot gas stream having a temperature above the melting point of the particles. The heat transfer from the gas to the particles generates the partial melting of the solid and the formation of droplets of melt material on the solid surface (sweating); the detachment of said droplets results in the purification of the solid. However, the concerned patent does not contain any example of application, and the yield of such process is not known.

With reference to the more general problem of separating a chemical product obtained in the crystalline form e.g. by crystallisation from solution or by crystallisation from the melt, the British patent GB-A-1322020 (Hoechst) discloses a process for the separation of isomer mixtures by crystallisation from the melt, followed by purification of the main isomer obtained in the crystal mass by the so-called "pressing" process. According to the disclosure, while separating the crystal mass from the residual melt by means of an ordinary filtration or centrifugation operation, the crystal mass may be "prepurified" - before undergoing the purification by pressing - by superficially melting the crystals by means of a flow of a warm inert gas.

Further, the British patent GB-A-956997 (Halcon) discloses a process for producing fresh or potable water from saline water by crystallisation of pure ice obtained by contacting the saline water with a liquid refrigerant, wherein a centrifugation operation is carried out, as usual, to separate the crystalline mass obtained from the residual mother liquor (brine, in this case). Also in this case, a superficial melting of the ice crystals is obtained in order to create a film of water between the ice crystal and the outer layer of mother liquor (brine), thereby facilitating the subsequent separation of saline water from the ice crystals by centrifugation.

Therefore, it is an object of the present invention to provide a new technology for the purification of chemical compounds, based on dry-sweating, which allows to overcome the drawbacks referred to in the foregoing, by providing in an extremely simple operation a high purification efficiency and a liquid residue with a high concentration of impurities.

To that aim it has been considered, according to the invention, that draining away the melt produced in the sweating operation, which is a critical factor for the process efficiency, cannot be readily obtained in the terrestrial gravitational field, while it may be carried out in a satisfactory way by subjecting the solid product to a centrifugal acceleration at least one order of magnitude higher than the gravity acceleration. By way of example, considering the detachment of a drop of ε-caprolactam - a chemical compound of great industrial importance - from the purified solid, it may be found that said detachment may occur only if the drop weight is several milligrams in the terrestrial gravitational field, while by operating with a centrifugal acceleration close to 100 x g the detachment of a drop having a 1000-fold smaller mass will be obtained. This results in a dramatic reduction of any difficulty in removing the melt from the solid, in comparison with the problems occurring in a conventional dry-sweating process.

Accordingly, the present invention specifically provides a process according to claim 1.

The melt material produced is rapidly drained away from the solid surface. Actually, the centrifugal force projects the melt onto the inner walls of the centrifugation apparatus, and from here the melt flows down the said walls and is collected through and outlet duct.

According to the invention, the solid compound to be purified (also referred to, further on, as the "raw product"), in pieces of a size preferably not greater than 5 cm, is placed into the rotary unit of a centrifugation apparatus with vertical or horizontal axis (said rotary unit having a perforated surface, with perforations suited to the size or grain size of the treated product); then the apparatus is made to work with an acceleration at least 10-fold the gravity acceleration (10 x g), while a hot inert gas is fed into the rotary unit, the nature of said gas being such as not to contaminate the product. Preferably, the solid product is heated in the shortest time possible to a temperature by no more than 10°C lower than the melting point of the corresponding pure solid product, and is maintained at said level to carry out the sweating process, for a period of time sufficient to obtain the desired sweating.

According to an alternative embodiment of the invention, the temperature of the solid is raised by means of the hot gas to a first fixed level, and is maintained at said level; then, it is further raised to one o more increasingly higher levels in subsequent steps, for a total period of time sufficient to obtain the desired sweating. Carrying out the process in more steps is an advisable method to control in the best way the amount of heat transferred from the gas to the solid and, consequently, to enhance the preferential melting of the most impure sections of the solid (namely, the sections containing the inclusions), this being a basic feature of the purification process. Actually, it is to be considered that as the purification proceeds the melting temperature of the solid increases, so that, in order to maintain a steady thermal flow from gas to solid in the course of the operation it is necessary to gradually increase the gas temperature.

As it is known, the first drops of melt produced in the dry-sweating are those containing the highest amount of impurities; as the treatment proceeds, the impurity concentration in the melt drops decreases. As a result, the rate of the purification process progressively falls. The melt should be limited to a mass percent not exceeding 20-30% by weight of the starting solid, both because for higher melt amounts the purity of the treated solid would not be appreciably increased, and because the process efficiency, in terms of percent amount of final solid obtained from the treatment, would be drastically reduced. In view of that, the treatment according to the invention is preferably carried out for a period of time suited to obtain the melting of no more than 20-30% by weight of the solid chemical product to be purified.

Since the factors determining the amount of heat transferred from the gas to the solid are the heat-transfer coefficient by forced convection between gas and solid and the temperature gradient between the gas and the solid surface, it is possible to obtain the same thermal flow by reducing the amount of gas and increasing the thermal gradient, i.e. the gas temperature, or, vice-versa, by increasing the amount of gas and reducing its temperature.

Preferably, at the end of the proper sweating phase the temperature of the hot gas is brought back to room temperature, while the gas is still fed to the centrifugation apparatus, thus accelerating the cooling of the purified solid before the final stop of the centrifugation apparatus. According to another preferred solution, before loading said centrifugation apparatus with the solid product to be purified, the interior of the apparatus is pre-heated with the same hot inert gas.

As noted before, the purification efficiency in the sweating process is usually measured by the parameter known as coefficient of distribution of impurities (k_{d}, equal to the ratio between the concentration of impurities of the solid after and before the treatment). The treatment concerned affords values of such parameter in some cases below 0.1, thus showing that it is possible to reduce the impurities content in the solid treated by one order of magnitude in one only process stage. By way of example, in one experimentally ascertained case wherein the melt mass of the residue amounts to 15% and the distribution coefficient is 0.2, there is obtained a concentration of the impurities in the melt equal to about 5.5 times the concentration in the solid to be treated, and about 28 times the concentration obtained in the purified solid product. Therefore, one only stage of the treatment based on the proposed technology allows to obtain, besides a good purification of the solid product, a residual melt having a high concentration of impurities. This second aspect appears to be quite advantageous if the above data are compared with the data given in the foregoing for the technology based on dynamic crystallization with the falling film technique and sweating (coefficient of distribution of the impurities equal to 0.34, with a concentration of impurities in the residue 2.3 times the concentration in the solid undergoing the treatment). As the corresponding value obtained for the latter ratio with the process according to the present invention is more than double, from the point of view of the process in its entirety there is achieved a dramatic reduction of the flow rate of the residue to be recycled to one of the plant sections upstream the one considered.

In order to make a comparison between the proposed technique and the conventional dynamic crystallization technique (without sweating) known as "solid-layer" - based on the formation of a crystalline layer on a cold surface - some tests have been carried out with a laboratory scale apparatus suited to carry out such technique. The apparatus consisted in an internally cooled steel cylinder, placed in the middle of a cylindrical vessel, where the melt product to be crystallized was kept under stirring. The operation procedure was the following: the sample to be purified, initially at the solid state, was placed in the vessel and was brought to the melt state, at a temperature some degrees higher than the initial melting point. When steady operation conditions where reached, the cylindrical crystallizer was immersed in the melt, and the crystallization of the melt started on the external surface of the cylinder. During the test the surface temperature of the cylindrical crystallizer was gradually reduced, according to the technique known as "progressive freezing". As it is known, this operating procedure allows to optimise the purification of the solid product. The test was stopped by withdrawing the cooled cylinder from the melt after some tens of minutes, namely when the growth rate of the crystalline layer had become extremely low. The final thickness of the crystalline layer was comprised between 2 and 4 mm.

On the same samples, the first one consisting in raw ε-caprolactam (CPL) and the second one consisting of raw benzyldimethylketal (BDK), both the treatment described above and the treatment of the present invention were carried out. The results thus obtained are compared in the following table.

**TABLE 1**

| PRODUCT | Crystalline layer formation (solid-layer technique) k_{d} | Dry-sweating in centrifuge according to the invention k_{d} |
|---|---|---|
| Raw CPL | 0.68 | 0.16 |
| Raw BDK | 0.35 | 0.14 |

The data reported in the table are averaged over at least three tests.

From the comparison, the improvements obtainable with the technique according to the invention are evident considering that the maximum value of the distribution coefficient of the impurities acceptable for an industrial application does not exceed 0.5, it may be observed that the purification of caprolactam tailings is not feasible with the conventional technique, while it is possible with the proposed technique. The purification of raw benryldimethylketal is possible with both techniques, although the technique based on dry-sweating affords a remarkably better performance.

The present invention is further described for illustrative purposes in the following examples; these are not to be intended as limiting anyhow the scope of the invention.

### EXAMPLE 1

### Purification of raw ε-caprolactam - Influence of the treatment temperature

The purification treatment according to the invention was applied to a sample of raw CPL coming from an industrial plant, having the following characteristics:

| | |
|---|---|
| Water content | 0.017 % by weight |
| Absorbance at 290 nm | 0.818 |

The above water content was measured with a Karl-Fisher analizer, while the absorbance value of a 50 wt.% water solution of CPL was measured with a spectrophotometer at a wavelength of 290 nm. The value of this parameter was taken as a measure of the organic impurities content.

The sample in solid form was crushed to pieces not exceeding 1 cm in size, in a laboratory mill. The solid particles were then sieved and two fractions were collected, the first one having a particle size comprised between 4 and 6.3 mm, and the second one between 1 and 4 mm. Of these fractions, the one having greater grain size underwent the following experimentation.

The purification tests with the technology according to the invention were carried out employing an apparatus based on laboratory centrifuge. The latter had vertical axis, a diameter of 120 mm and a capacity of 250 ml, and could work at a rotary speed comprised between 1000 and 10000 r.p.m., corresponding respectively to "g" values of 67.3 and 6730. In order to avoid any loss of solid through the basket perforations, the basket surface was lined with filter paper having openings of 30 µm.

Each test was carried out according to the following general procedure:
1. the inner section of the centrifuge was pre-heated with a stream of hot nitrogen, up to a temperature about 5°C below the temperature set for the test;
2. the centrifuge basket was loaded with 10 g of the solid to be treated;
3. the rotation of the centrifuge basket was started, at 5000 r.p.m., and at the same time nitrogen at the fixed temperature started to be fed to the inner section of the centrifuge, with a flow rate of 1750 Nl/h;
4. as the test proceeded, the centrifuge temperature, evaluated as the arithmetic mean of the temperature inside and outside the inner basket, progressively increased;
5. once the fixed temperature was reached, the treatment time count started; this step was optionally repeated at a higher temperature in a subsequent step;
6. at the end of the period of time fixed for the treatment, the heating of the nitrogen stream was stopped, and the rapid cooling of the solid particles started, by operating with gas at room temperature;
7. as soon as the centrifuge temperature reached a level about 20°C lower than the treatment temperature, the test was stopped by discontinuing the gas feed and by stopping the basket rotation;
8. the basket was taken out from the centrifuge and the solid was collected, weighed and analyzed for the required measures.

The following table summarizes the operating conditions and the results of the tests carried out in order to evaluate the influence of the treatment temperature on the process performance.

**TABLE 2**

| Test No. | Time (1^{st} step+2^{nd} step) min. | Temperature (1^{st} step/2^{nd} step) °C | Mass reduction % | k_{d} |
|---|---|---|---|---|
| 1A | 10+10 | 63/69 | 14.2 | 0.45 |
| 1B | 10+10 | 63/69.5 | 20.0 | 0.39 |
| 1C | 10+10 | 63/71 | 38.3 | 0.30 |

On the grounds of what set forth on the foregoing, the coefficient of distribution of the impurities was held to be represented by the ratio between the absorbance of the treated solid and the absorbance of the starting solid. Throughout the test, the absorbance was measured at a wavelength of 290 nm.

The results obtained show a remarkable influence of the temperature, both on the amount of melt mass and on the solid purification. A temperature increase, while being always beneficial to the purification, may involve - as in the case of the test 1C - an excessive melting of the solid, thus reducing the final yield of the operation. Among the series of tests reported in Table 2, the best operation condition appears to be the one corresponding to test 1B, since it allows to obtain at the same time a good purification and a limited melting of the treated solid.

### EXAMPLE 2

### Purification of raw ε-caprolactam - Influence of the solid material size

By operating according to the same procedure of the previous example, two purification tests on samples of CPL tailings having different particle size were carried out, the two ranges of particle size being, respectively, 1-4 mm and 4-6.3 mm.

During each test the average temperature of the gas in the centrifuge was maintained at the fixed level of 68 °C.

The following table reports the results of 4 tests, carried out on samples of different grain size and for different treatment times.

**TABLE 3**

| Test No. | Grain size mm | Time min. | Mass reduction % | k_{d} |
|---|---|---|---|---|
| 2A | 4-6.4 | 30 | 13.9 | 0.47 |
| 2B | 1-4 | 30 | 29.7 | 0.16 |
| 2C | 1-4 | 20 | 20.2 | 0.16 |
| 2D | 1-4 | 15 | 10.8 | 0.37 |

From the above data it appears that the reduction in the grain size of the solid to be treated results in a quite better performance of the process. Firstly, it is possible to reach very high purity values in the final solid product. Actually, the distribution coefficient 0.16 derives from an absorbance value of 0.131, which is very close to the absorbance of the commercial product (i.e., 0.090). In addition, if the result of the test 2A is compared with the results of the tests 2C and 2D, it appears that with a smaller grain size it is possible to reduce the treatment time from 30 minutes to less than 20 minutes, while obtaining an equivalent amount of melt mass and a better purification.

The better efficiency of the treatment is due to the fact that, for a given migration speed, in order to reach the solid surface and be expelled therefrom the inclusions have to cover a path which is directly proportional to the particle size. As a result, the smallest particles, having an average size of 2.5 mm, require a migration time for the inclusions expulsion about one half the time required for the largest solid particles.

On the other hand, the higher melting rate of the smallest particles is caused by the great amount of heat being transferred to the particles from the gas, in view of a higher surface/volume ratio.

### EXAMPLE 3

### Purification of raw benzyldimethylketal

The treatment according to the invention was applied to tailings of the industrial production of benzyldimethylketal (BDK), a product of fine organic chemistry employed as a polymerization promoter.

The starting product, a solid in pieces of remarkable size, was ground, and the solid particles thus obtained were sieved and divided into two fractions of size comprised between 1 and 4 mm and between 4 and 6.4 mm respectively. Of these fractions, the one having greater grain size underwent the following experimentation.

Pure BDK has a melting point of 66.5 °C. Since the purpose of the treatment is to obtain a purification of the starting solid by means of a partial melting involving no more than 20% of the same solid, the temperature of the hot gas employed did not exceed 65°C. The operating procedure employed for each test was the same as that described in Example 1, except for the fact that, in the case of BDK, air was used instead of nitrogen.

A first series of 2 tests was carried out to study the treatment efficiency, employing air at a the temperature of 64°C and a treatment time of 10 minutes. The following table reports a comparison between the purity of the starting product and the average purity obtained in the two concerned tests and, finally, the purity of the commercial product, for comparison.

**TABLE 4**

| Product | Purity wt. % | Benzyl wt. % | Transmittance at 425 nm, % | Transmittance at 500 nm, % |
|---|---|---|---|---|
| BDK tailings | 97.3 | 0.9 | 70.3 | |
| Purified | 99.5 | 0.1 | 94.7 | 98.0 |
| Commercial Product | ≥99.5 | | ≥97.0 | ≥98.0 |

A comparison between the details given for the purified product and for the commercial product show that the purification obtained is similar to the purification level of the commercial product both as concerns the percent purity and as concerns the transmittance at 500 nm, while there is still some discrepancy as concerns the transmittance at 425 nm. The treatment efficiency is also shown by the analysis of one of the most important impurities present in the BDK tailings, i.e. benzyl, the final content of which upon the treatment is about 1/10 of the initial content.

The mass reduction obtained in the two tests is about 16.5%, so that the impurity concentration in the process residue may be considered to be about 14%, with an approximate, 5-fold increase with respect to the starting product.

### EXAMPLE 4

### Purification of raw benzyldimethylketal - Influence of the treatment time

Following the same procedure described in Example 3, a series of 3 tests were carried out in order to evaluate the influence of the treatment time on the purification and on the mass reduction due to melting.

Such tests were performed with an air stream at the temperature of 64°C, and with a grain size of the solids comprised between 4 and 6.4 mm.

With regard to the purification, the results were evaluated in terms of transmittance at 425 nm, which represents the most restrictive requirement. The results of the experimentation are summarized in the following table.

**TABLE 5**

| Test No. | Time min. | Mass reduction % | Transmittance at 425 nm % |
|---|---|---|---|
| 4A | 5 | 12.4 | 90.4 |
| 4B | 10 | 15.4 | 92.4 |
| 4C | 20 | 18.2 | 94.6 |

It may be noted that the purification and the melt mass are proportional to the treatment time of the test. In all tests performed, a good purification was obtained with respect to the starting product, having a transmittance of 70.3%. Also, a limited mass reduction was obtained, which affords a remarkable concentration of the impurities in the residue.

### EXAMPLE 5

### Purification of raw benzyldimethylketal - Influence of the solid material size

In order to ascertain the effect of the particle size on the purification efficiency, a series of tests following the same procedure of Example 3 was carried out, employing solid particles of raw BDK having a particle size comprised between 1 and 4 mm. The temperature of the feed of hot air was maintained at 64 °C.

The results of this set of tests are reported in the following table, in comparison with the results already presented in Example 3 for the greater grain size.

**TABLE 6**

| Test No. | Particle size mm | Time min. | Mass reduction % | Transmittance at 425 nm % |
|---|---|---|---|---|
| 5A | 1-4 | 10 | 16.0 | 95.2 |
| 5B | 1-4 | 20 | 16.1 | 97.0 |
| 4B | 4-6.4 | 10 | 15.4 | 92.4 |
| 4C | 4-6.4 | 20 | 18.2 | 94.6 |

From a comparison of the above data it may be inferred that the reduction of the solid particle size is quite advantageous for the purpose of the purification of raw BDK, as it is for raw CPL (see Example 2). This is a consequence of the shorter path that the inclusions present in the solid have to cover to reach the surface thereof, and be expelled from the solid. On the other hand, the reduction of the particle size does not result in any appreciable variation of the amount of the melt mass.

Finally, it is to be noticed that the treatment 5B allowed to obtain a transmittance at 425 nm of the purified product equal to the minimum transmittance admitted for the commercial product.

The present invention has been disclosed with particular reference to some specific embodiments thereof, but it should be understood that modifications and changes may be made by the persons skilled in the art without departing from the scope of the invention as defined in the appended claims.

## Claims

1. A process for purifying raw solid chemical products obtained by crystallization and containing impurity inclusions by dry-sweating, comprising: a) centrifuging the raw solid chemical product to be purified, in divided form in pieces of a size not greater than 5 cm, in a rotary centrifugation apparatus having an inner basket permeable to melt material only, in which apparatus a hot inert gas is fed at the same time, said gas having a temperature and a flow rate and being fed for a total period of time suitable to heat the said solid product to a temperature slightly above the equilibrium temperature of the pure solid in contact with the melt mixture present as the inclusion so as to obtain the sweating of the row solid chemical product that is, the partial melting with simultaneous purification, wherein the melting occurs around the impurity inclusions; and b) recovering, at the end of said centrifuging, the purified solid product from the said basket, while the melt product containing a higher concentration of impurities is collected in the external container of the centrifugation apparatus.

2. A process according to claim 1, wherein said treatment in a centrifugation apparatus is carried out at not less than 10 x g.

3. A process according to any one of claims 1-2, wherein the said solid product is heated to a temperature by no more than 10°C lower than the melting point of the corresponding pure solid product.

4. A process according to any one of claims 1-3, wherein the temperature of said solid is raised, by means of said hot gas, to the fixed level, and is then maintained at said level for a period of time sufficient to obtain the desired sweating.

5. A process according to any one of claims 1-3, wherein the temperature of said solid is raised, by means of said hot gas, to the fixed level, and is then further raised to one or more increasingly higher levels in subsequent steps, for a total period of time sufficient to obtain the desired sweating.

6. The process according to claims 4 or 5, wherein the said period of time is such as to obtain the melting of no more than 20-30% by weight of said solid chemical product to be purified.

7. A process according to any one of claims 4-6, wherein at the end of said period of time of treatment, the temperature of said hot gas is brought back to room temperature, while the gas is still fed to the centrifugation apparatus, thus accelerating the cooling of said purified solid before the final stop of the centrifugation apparatus.

8. The process according to any one of claims 1-7, wherein before loading said centrifugation apparatus with said solid product to be purified the interior of the centrifugation apparatus is pre-heated with said hot inert gas.

9. A process according to any one of claims 2-8, wherein the said solid chemical product to be purified is in pieces of a size not greater than 1 cm.

10. The process according to any one of the previous claims, for the purification of caprolactam by dry-sweating.

11. The process according to any one of the previous claims, for the purification of benzyldimethylketal by dry-sweating.

## Patentansprüche

1. Verfahren zur Reinigung fester chemischer Rohprodukte, welche durch Kristallisation erhalten wurden und Einschlüsse von Verunreinigungen enthalten, durch trockenes Ausschwitzen, umfassend: a) Zentrifugieren des zu reinigenden festen chemischen Rohprodukts, in Stücke einer Größe von nicht mehr als 5 cm aufgeteilt, in einer Rotationszentrifugationsvorrichtung mit einem inneren Korb, der nur für geschmolzenes Material durchlässig ist, welcher Vorrichtung gleichzeitig ein heißes Inertgas zugeführt wird, wobei das Gas eine geeignete Temperatur und Durchflussrate aufweist und für einen geeigneten Gesamtzeitraum zugeführt wird, um das feste Produkt auf eine Temperatur zu erwärmen, die leicht über der Gleichgewichtstemperatur des reinen Feststoffs in Kontakt mit der Schmelzmischung, die als Einschluss vorliegt, liegt, um das Ausschwitzen des festen chemischen Rohprodukts zu erzielen, d.h., das partielle Schmelzen mit gleichzeitiger Reinigung, wobei das Schmelzen um die Einschlüsse von Verunreinigungen stattfindet, und b) Gewinnen des gereinigten festen Produkts am Ende der Zentrifugation aus dem Korb, wobei das Schmelzprodukt, das eine höhere Konzentration an Verunreinigungen enthält, in dem äußeren Behälter der Zentrifugationsvorrichtung gesammelt wird.

2. Verfahren nach Anspruch 1, wobei die Behandlung in einer Zentrifugationsvorrichtung bei nicht weniger als 10 x g durchgeführt wird.

3. Verfahren nach irgendeinem der Ansprüche 1 bis 2, wobei das feste Produkt auf eine Temperatur von nicht mehr als 10°C niedriger als der Schmelzpunkt des entsprechenden reinen festen Produkts erwärmt wird.

4. Verfahren nach irgendeinem der Ansprüche 1 bis 3, wobei die Temperatur des Feststoffs mittels des heißen Gases auf das festgelegte Niveau erhöht wird und dann bei diesem Niveau für einen ausreichenden Zeitraum gehalten wird, um das gewünschte Ausschwitzen zu erzielen.

5. Verfahren nach irgendeinem der Ansprüche 1 bis 3, wobei die Temperatur des Feststoffs mittels des heißen Gases auf das festgelegte Niveau erhöht wird und dann weiter auf ein oder mehrere zunehmend höhere Niveaus in nachfolgenden Schritten erhöht wird, für einen Gesamtzeitraum, der ausreicht, um das gewünschte Ausschwitzen zu erzielen.

6. Verfahren nach den Ansprüchen 4 oder 5, wobei der Zeitraum derart ist, dass das Schmelzen von nicht mehr als 20 bis 30 Gew.-% des zu reinigenden festen chemischen Produkts erhalten wird.

7. Verfahren nach irgendeinem der Ansprüche 4 bis 6, wobei am Ende des genannten Behandlungszeitraums die Temperatur des heißen Gases auf Raumtemperatur zurückgebracht wird, während das Gas immer noch der Zentrifugationsvorrichtung zugeführt wird, und somit die Abkühlung des gereinigten Feststoffs vor dem endgültigen Abstoppen der Zentrifugationsvorrichtung beschleunigt wird.

8. Verfahren nach irgendeinem der Ansprüche 1 bis 7, wobei vor dem Beladen der Zentrifugationsvorrichtung mit dem zu reinigenden festen Produkt das Innere der Zentrifugationsvorrichtung mit dem heißen Inertgas vorgewärmt wird.

9. Verfahren nach irgendeinem der Ansprüche 2 bis 8, wobei das zu reinigende feste chemische Produkt in Stücken einer Größe von nicht mehr als 1 cm vorliegt.

10. Verfahren nach irgendeinem der vorhergehenden Ansprüche zur Reinigung von Caprolactam durch trockenes Ausschwitzen.

11. Verfahren nach irgendeinem der vorhergehenden Ansprüche zur Reinigung von Benzyldimethylketal durch trockenes Ausschwitzen.

## Revendications

1. Procédé de purification de produits chimiques solides bruts obtenus par cristallisation et contenant des inclusions d'impuretés par ressuage à sec, qui comprend : a) la centrifugation du produit chimique solide brut à purifier, sous forme divisée en morceaux dont la taille n'excéde pas 5 cm, dans un appareil de centrifugation rotatif présentant un panier interne perméable au matériau fondu uniquement, appareil dans lequel on amène dans le même temps un gaz chaud inerte, ledit gaz ayant une température et un débit et étant amené sur un intervalle de temps total approprié pour chauffer ledit produit solide à une température légèrement supérieure à la température d'équilibre du solide pur en contact avec le mélange fondu présent sous forme d'inclusions, de façon à obtenir un ressuage du produit chimique solide brut, à savoir la fusion partielle avec purification simultanée, la fusion s'effectuant autour des inclusions d'impuretés ; et b) la récupération, à la fin de ladite centrifugation, du produit solide purifié depuis ledit panier, alors que le produit fondu contenant une concentration supérieure en impuretés est recueilli dans le conteneur externe de l'appareil de centrifugation.

2. Procédé selon la revendication 1, dans lequel ledit traitement dans un appareil de centrifugation est réalisé à au moins 10 x g.

3. Procédé selon l'une quelconque des revendications 1-2, dans lequel ledit produit solide est chauffé à une température n'excédant pas 10 °C de moins que le point de fusion du produit solide pur correspondant.

4. Procédé selon l'une quelconque des revendications 1-3, dans lequel la température dudit solide est élevée, au moyen dudit gaz chaud, au niveau fixé, et est maintenue à ce dit niveau pendant une durée suffisante pour obtenir le ressuage désiré.

5. Procédé selon l'une quelconque des revendications 1-3, dans lequel la température dudit solide est élevée, au moyen dudit gaz chaud, au niveau fixé, puis est à nouveau augmentée vers un ou plusieurs niveaux toujours plus élevés lors d'étapes ultérieures, pendant une durée totale suffisante pour obtenir le ressuage désiré.

6. Procédé selon la revendication 4 ou 5, dans lequel ladite période est telle que l'on obtient la fusion au maximum de 20 à 30 % en poids dudit produit chimique solide à purifier.

7. Procédé selon l'une quelconque des revendications 4-6, dans lequel, à la fin de ladite période de traitement, la température dudit gaz chaud est ramenée à la température ambiante, alors que le gaz est toujours amené vers l'appareil de centrifugation, accélérant alors le refroidissement dudit solide purifié avant l'arrêt final de l'appareil de centrifugation.

8. Procédé selon l'une quelconque des revendications 1-7, dans lequel l'intérieur de l'appareil de centrifugation est pré-chauffé avec ledit gaz inerte chaud avant le chargement dudit appareil de centrifugation avec ledit produit solide à purifier.

9. Procédé selon l'une quelconque des revendications 2-8, dans lequel ledit produit chimique solide à purifier se présente en morceaux dont la taille n'excéde pas 1 cm.

10. Procédé selon l'une quelconque des revendications précédentes pour la purification du caprolactame par ressuage à sec.

11. Procédé selon l'une quelconque des revendications précédentes pour la purification du benzyldiméthylcétal par ressuage à sec.
